(19) Europäisches Patentamt
European Patent Office
Office européen des brevets

(11) **EP 3 845 124 A1**

(12) **EUROPEAN PATENT APPLICATION**

(43) Date of publication:
**07.07.2021 Bulletin 2021/27**

(51) Int Cl.:
***A61B 5/055*** (2006.01)    ***A61B 5/02*** (2006.01)

(21) Application number: **20150306.7**

(22) Date of filing: **06.01.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **Koninklijke Philips N.V.**
**5656 AG Eindhoven (NL)**

(72) Inventors:
• **STEUNEBRINK, Tim Patrick**
**5656 AE Eindhoven (NL)**

• **VERNOOIJ, Carlijn Andrea**
**5656 AE Eindhoven (NL)**
• **PEETERS, Wouter Herman**
**5656 AE Eindhoven (NL)**
• **DOODEMAN, Gerardus Johannes Nicolaas**
**5656 AE Eindhoven (NL)**
• **ROCK, Joseph Ernest**
**5656 AE Eindhoven (NL)**

(74) Representative: **Philips Intellectual Property & Standards**
**High Tech Campus 5**
**5656 AE Eindhoven (NL)**

(54) **INDUCTIVE SENSING SYSTEM AND METHOD**

(57)    An inductive sensing system (8) is for detecting bleeding (e.g. blood pools) in one or more regions of the body. The system comprises a resonator circuit (10) having at least one antenna (12) which is driven with an oscillatory drive signal to cause generation of electromagnetic signals for application to a body. The signals induce eddy currents in the body which generate secondary EM signals returned from the body. These interact with the resonator circuit by adding an additional component of inductance to the circuit. This inductance component varies depending upon the conductivity of the fluid in which the eddy current is induced. Blood has a different conductivity to other body fluids. The system is configured to detect presence of abnormal accumulations pf blood based on the additional inductance component. The system generates a data output representative of the determination.

FIG. 2

EP 3 845 124 A1

**Description**

FIELD OF THE INVENTION

This invention relates to an inductive sensing system and method, in particular for sensing presence of blood pools in one or more regions of the body.

BACKGROUND OF THE INVENTION

Internal bleeding is a major acute condition and requires prompt treatment to prevent rapid deterioration. Two common kinds of internal bleeding include intracerebral hemorrhage and abdominal bleeding.

Intracerebral hemorrhage (ICH) requires treatment extremely quickly; time is the critical factor for patient outcome. A major problem is that ICH is often not immediately recognized in the prehospital setting (e.g. in the community, a patient's home or in the ambulance). It is often only diagnosed much later, inside the hospital, or after the patient has begun to decompensate. Proper diagnosis earlier in the pre-hospital setting would facilitate transportation to an appropriate facility and, if the patient was not suffering from an ICH, permit timely treatment to be applied, preventing serious deterioration and also improving the preparation and planning of the correct treatment once the patient arrives at the hospital. Furthermore, currently, the only known methods for ICH diagnosis are expensive, in-hospital scans (CT scan or MRI scan), the results of which are evaluated by a neuroradiologist. Although accurate, these scans are expensive and time consuming, and are also unsuitable for pre-hospital settings.

Hence, there remains need for a fast and relatively compact means to reliably diagnose intracerebral hemorrhage in a prehospital setting.

Abdominal pain is one of the most common clinical presentations in an emergency department. Diagnosis of blunt abdominal injury is critical for positive patient outcome. An undiagnosed abdominal bleeding can have serious consequences for the patient, and can even be fatal. In current practice, examination of intra-abdominal bleeding is performed via manual palpation or use of ultrasound as part of the Focused Assessment with Sonography for Trauma (FAST) approach. Although fast, these methods are not accurate. Manual palpation requires expertise of the medical specialist to reliably scope for abdominal sensitivities. Furthermore, even with the input of a medical expert, only the liver and kidneys may be palpable while other organs may be more problematic for diagnosis. Furthermore, the patient is required to be conscious to indicate tenderness which may not always be the case in a triaging situation.

FAST technology has made significant advances in recent years, and has been used for detecting abdominal bleeding. However this modality is also not fully accurate. Acquisition of diagnostic quality ultrasound images depends upon the skill of the operator, and hence is not always reliable. Several diagnostic errors frequently occur due to the low sensitivity of ultrasound to intra-abdominal bleeding. Visceral bleeding in particular is difficult or even impossible to detect using ultrasound, meaning a significant false-negative rate within ultrasound scans. Furthermore, the human interpretation of ultrasonic output poses the risk of medical errors.

The most accurate method, as with ICH, remains the use of CT or MRI scans. However, tomography scans that are not necessary are also not beneficial to the patient (exposing the patient to unnecessary radiation and extending their stay in hospital) and also consume valuable resources for the hospital. Accurate and timely assessment and triaging of trauma patients for potential internal bleeding is therefore required to meet the demands of both the patient and the emergency department.

There remains therefore a need for an accurate and fast method for detecting abdominal bleeding of patients entering the emergency department.

Improved means for detecting internal bleeding able to remedy one of more of the above deficiencies would therefore be of advantage.

SUMMARY OF THE INVENTION

The invention is defined by the claims.

According to examples in accordance with an aspect of the invention, there is provided an inductive sensing system arranged for sensing electromagnetic signals returned from a body responsive to application of electromagnetic excitation signals to said body, the system adapted to detect presence of bleeding in one or more regions of the body, the system comprising:

a resonator circuit comprising at least one loop antenna, and an electronic signal generator coupled to the antenna, for driving the antenna with a drive signal to cause it to generate the electromagnetic excitation signals; and
a signal sensing means arranged for sensing, simultaneously with signal generation, said returned signals from the body based on detecting a measure indicative of an additional inductance component added to the antenna of the resonator circuit by said returned signals;
the system configured to:

determine presence or absence of accumulations of blood based on the detected measure of the additional inductance component, and
generate a data output indicative of a result of the determination.

The system is for detecting (abnormal) blood pools for example, i.e. collections or accumulations of blood.

The system may comprise a controller or processor configured to perform said determining and generating steps.

Embodiments of the invention are hence based on use of inductive sensing for detecting bleeding.

In accordance with one set of embodiments, the determining the presence or absence of blood accumulations may be based on a pre-defined threshold, the threshold associated with presence of bleeding or blood accumulation in at least one of said one or more regions of the body. However, use of a threshold represents just one example. Alternative approaches include for instance use of an algorithmic model which is configured to determine presence or absence of blood in one or more particular regions based on the detected additional inductance component. Other alternative approaches might include use for instance of artificial intelligence or machine learning algorithms, for instance trained for classifying between normal levels of blood in a region and abnormal pooling or accumulation of blood in a region. For example, a machine learning algorithm might be trained with training data, the training data comprising different additional inductance component signals for different regions, each labelled as to whether it corresponds to a normal blood presence or to abnormal accumulation or pooling of blood.

Inductive sensing is based on generation of a primary alternating magnetic field via a primary antenna loop, which leads to the induction of eddy currents and a consequent secondary magnetic field in conductive material or tissue within the primary magnetic field. Interaction of the secondary magnetic field with the primary loop or the primary magnetic field can be used to detect features of probed bodies, in particular those comprising conductive tissue or material, e.g. a water or other fluid content.

In particular, this field interaction leads to changes in the detectable electrical characteristics of the current running through the antenna coil. For example, the current frequency can be changed, and/or the current amplitude can be dampened.

In inductive sensing, a signal generator (such as an oscillator) is connected to a loop antenna. The oscillator is an amplifier, typically consisting of one or more transistors, which induces a resonant state in a coupled circuit, in combination with an inductance source and capacitance source. The inductance is provided by the loop antenna, while the capacitance is provided by an optional capacitor component placed in parallel to the loop, together with parasitic capacitances of the loop with itself and its environment, and the oscillator parasitic capacitances. The total system is called the resonator.

The secondary magnetic field generated by the body has the effect at the antenna loop of adding an additional complex inductance to the resonator circuit, which thereby leads to detectable changes in the circuit current. The real part of the additional inductance is detectable for example as changes in frequency of the oscillator circuit current. The imaginary part of the additional inductance is detectable for example as changes in amplitude of the oscillator circuit current (or voltage).

Inductive sensing can also be used to distinguish between different fluids in a material. The electromagnetic signal response from different fluids varies depending upon their conductivity which in turn varies depending upon their physical properties. This provides a means for detecting presence of pools of blood at locations in the body where normally there should be no blood, or there should only be smaller amounts of blood. For example, in the intracerebral region, in a healthy patient, there should be a layer only of cerebrospinal fluid (CSF), plus normal blood flow. In the case of intracerebral hemorrhage, pools or accumulations of blood are present in addition or instead due to abnormal bleeding. Similarly, for the abdominal region, for a healthy patient, there should be only abdominal or visceral fluid present, in additional to normal blood flow through arteries, veins and capillaries in the region. Abdominal or visceral fluid has a different conductivity to blood. Pools or accumulations of blood will result in a different returned inductive signal than normal blood flow and normal abdominal and visceral fluid. Thus by measuring the inductive signal response at the relevant anatomical region, it can be determined whether abnormal accumulation of blood is present or only the normal fluids.

For example, according to one or more embodiments, the system may be configured for detecting presence of accumulation of blood in an intracerebral region of the body. The system in this case may be configured for detecting occurrence of intracerebral hemorrhage based on the blood detection.

For example a signal threshold may be defined, this threshold known to be associated with presence of bleeding in the intracerebral region or the abdominal region as appropriate.

According to one or more embodiments, the system may be configured for detecting presence of accumulation of blood in an abdominal region of the body. The system in this case may be configured to detect presence of abdominal bleeding based on the blood detection.

For example, a signal threshold may be defined, wherein this may be a threshold known to be associated with presence of bleeding in the intracerebral region or the abdominal region as appropriate.

In accordance with one or more embodiments, the system may be switchable between at least two detection modes:

a first detection mode in which the system is configured for detecting presence of blood accumulation in a cranial region of the body; and

a second detection mode in which the system is configured for detecting presence of blood accumulation in an abdominal region of the body.

There are different possible configurations for the resonator circuit and antenna. In particular, the resonator can be provided with only a single antenna or with a plurality of antennas. In each case, preferably each antenna comprises a single loop (single turn or winding) only.

It has been found that where the resonator circuit is provided with a single antenna only, the largest difference in inductive response between blood and non-blood body fluid is exhibited in the real component of the additional inductance component added the resonator circuit.

Hence, where a single antenna is provided, the signal sensing means may be configured to detect a real component of said additional inductance component added to the resonator circuit. This however is not essential, and the method also works for example if the imaginary component of the additional inductance is detected instead.

In particular embodiments, detection of presence or absence of accumulations of blood may comprise comparing the detected real component of the additional inductance with a real inductance threshold associated with presence of blood accumulation.

In accordance with an alternative set of embodiments, the resonator circuit may comprise at least two single loop antennas, and wherein the system is configured to detect an additional inductance component added to each one of the antennas by said returned signals from the body.

Use of more than one antenna may improve accuracy or reliability of blood detection. In particular, measurement results with more than one loop antenna are less sensitive to small changes in the tissue layers between the loops and the fluid. For example, if the fat layer of the abdominal wall differs from patient to patient, the additional inductance measured with a single loop may change due to the increased or decreased distance of the layer of (accumulated) blood to the loop. With a multi-loop setup, the measurement result of both loops will change but the ratio between the two measurement results will remain approximately stable. Therefore, the accuracy of determining blood presence increases.

In the multiple loop setup, according to certain examples, the system may be further configured to determine a ratio between the additional inductance components added to each of the two antennas, and to determine presence or absence of blood accumulation based on this ratio.

Ratio in this context may mean a quotient of the reflected inductance components for the two loops.

In particular examples, the system may be configured to compare said ratio with a pre-defined threshold to determine presence or absence of blood accumulation.

Hence in this example, a threshold is pre-defined, wherein the threshold is a threshold pertaining to the ratio of values between the two loops.

In other examples, detection of blood accumulation may be based on a difference between the additional inductance components added to each of the two antennas. For example, this difference may be compared to a pre-defined threshold to determine presence or absence of blood accumulation.

Thus more generally, detection of blood accumulation may be based on comparative values of the inductance components added to each of the two antennas.

It has been found that for a multi-loop setup in which a ratio between the inductance added to each loop is used, the largest difference in the inductance signal between blood and other fluids is found for the imaginary components of the inductance. Hence, in advantageous examples, the signal processing means may be configured in these cases to extract an imaginary part of the additional inductance component added to each of the antennas and to determine a ratio between said imaginary components. This may provide more accurate or reliable detection results.

However, use of the imaginary component is not essential and the method still works with use for example of the real component of the additional inductance component added to each antenna.

Although an example with two antennas is mentioned, the resonator circuit may comprise more than two antennas.

Where two or more loops are provided, there are different options for their confi gurati on.

The two loop antennas may be arranged concentrically with one another.

Additionally or alternatively, the two loop antennas may be mounted axially offset with respect to one another. Axially offset in this context means offset in a direction perpendicular a plane defined by at least one of the antenna loops (the plane in which the antenna lies).

Additionally or alternatively, the signal generator may be configured to drive the two antennas with drive signals of different respective AC frequencies.

In accordance with one or more embodiments, the system may comprise a support structure, the at least one loop antenna being mounted to the support structure.

The support structure is for example for holding the one or more antennas in a fixed position. Where there are two antennas, it may hold them in a fixed position relative to one another. It may be or include a frame structure. It be or include a housing structure. Where a housing is provided, the housing may be arranged to house the resonator circuit including the antenna(s).

In accordance with one or more embodiments, the sensing system may comprise a handheld probe unit. The handheld probe unit may comprise, e.g. contain or house, at the least the resonator circuit.

A handheld probe configuration permits the system to be used for blood detection at a range of different regions of the body, including the head or the abdomen, as required. The system may be switchable between different detection modes for instance, for example as described above, for use with different regions of the body.

In accordance with one or more embodiments, the system may comprise a head wearable cap, at least one antenna being mounted to the cap. The mounting may be such that, when the cap is worn, at least one antenna is held in fixed relation to a surface of the head.

In preferred examples, preferably the system may comprise a plurality of antennas mounted to the cap at different positions, so as to be held at different locations about the head when the cap is worn.

Such a cap provides an efficient arrangement for detecting bleeding in the head region for example for detecting intracerebral hemorrhage. Providing multiple antennas at different locations around the cap means that bleeding across the whole area of the head can be monitored, and for example a map developed of the signals detected around the head.

In some examples, the cap may be formed of a flexible material so as to be retained around the head when worn. However this is not essential.

In accordance with one or more embodiments, the system may comprise a (e.g. textile) garment or sheet for covering at least one of said one or more regions of the body, the at least one antenna being mounted to the garment or sheet.

In preferred embodiments, the system may comprise a plurality of antennas, mounted to the garment or sheet at different positions.

According to specific examples, the garment or sheet may comprise a blanket or a vest.

A garment or sheet may be particularly advantageous for detecting bleeding in an abdominal area. For example, a blanket may be laid across the abdomen of the patient, simultaneously providing an insulation function, and also enabling inductive detection of any blood pooling in the abdomen.

According to one more advantageous embodiments, the garment or sheet may comprise a visual indictor means, the system configured to control the visual indicator means to provide a visual indication of the determination of presence or absence of blood. The visual indicator may for example comprise one or more light sources, for example red and green lights, for example indicating presence of bleeding and absence of bleeding respectively. This feature can additionally or alternatively be applied to the head wearable cap mentioned above to provide visual indication of presence of bleeding.

Examples in accordance with a further aspect of the invention provide an inductive sensing method for detecting presence of bleeding in one or more regions of a patient's body, the method based on sensing electromagnetic signals returned from the body responsive to application of electromagnetic excitation signals to said body, the method comprising:

driving at least one loop antenna of a resonator circuit with a drive signal to cause it to generate the electromagnetic excitation signals;
sensing, simultaneously with the signal generation, said returned signals from the body based on detecting a measure indicative of an additional inductance component added to the antenna of the resonator circuit by said returned signals;
determining presence or absence of blood accumulation based on the detected measure of the additional inductance component; and
generating a data output indicative of a result of the determination.

In particular embodiments, the determining presence or absence of blood accumulations or pools may be based on a pre-defined threshold, the threshold associated with presence of (abnormal) accumulation of blood in at least one of said one or more regions of the body.

The method may be for detecting presence of blood accumulations in an intracerebral region. The method may be for detecting presence of blood accumulation in an abdominal region. The method may further comprise determining, based on blood detection, occurrence of either intracerebral hemorrhage or abdominal bleeding.

For example, the method may comprise positioning the at least one antenna of the resonator circuit for applying electromagnetic excitation signals to the head region or the abdominal region, and receiving returned electromagnetic signals from the head region or abdominal region.

Where a threshold is used for making the detection, the threshold may be a threshold known to be associated with presence of bleeding in the cranial region or the abdominal region as appropriate.

Examples in accordance with a further aspect of the invention may provide a computer program product comprising code means configured, when executed on a processor, the processor bring operatively coupled to a

> a resonator circuit comprising at least one loop antenna, and an electronic signal generator coupled to the antenna, for driving the antenna with a drive signal to cause it to generate the electromagnetic excitation signals, and
> a signal sensing means arranged for sensing, simultaneously with signal generation, said returned signals from the body based on detecting a measure indicative of an additional inductance component added to the antenna of the resonator circuit by said returned signals

to cause the processor to perform an inductive sensing method in accordance with any embodiment outlined above or described below, or in accordance with any claim of this application.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

> Fig. 1 schematically illustrates eddy current induction within a body to which the system is applied;
> Fig. 2 shows a schematic block diagram of components of an example system in accordance with one or more embodiments;
> Fig. 3 illustrates an example arrangement comprising two antennas;
> Figs. 4 and 5 show graphs illustrating comparative inductance responses for blood and CSF when using a single antenna, for imaginary and real components of inductance respectively;
> Figs. 6 and 7 show graphs illustrating comparative inductance responses for blood and CSF when using two antennas, for imaginary and real components of inductance respectively;
> Figs. 8 and 9 schematically illustrate a head-worn cap unit comprising multiple antennas mounted thereto;
> Fig. 10 shows closer views of the attachment means for the antennas to the head cap;
> Fig. 11 schematically illustrates an example sheet or garment comprising a plurality of antennas mounted thereto; and

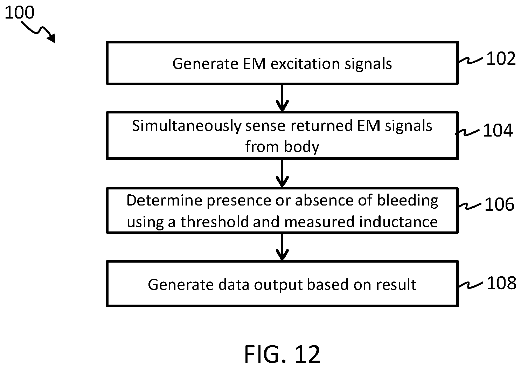

> Fig. 12 outlines in block diagram form an example method in accordance with one or more embodiments.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an inductive sensing system for detecting bleeding (e.g. blood pools) in one or more regions of the body. The system comprises a resonator circuit having at least one antenna which is driven with an oscillatory drive signal to cause generation of electromagnetic signals for application to a body. The signals induce eddy currents in the body which generate secondary EM signals returned from the body. These interact with the resonator circuit by adding an additional component of inductance to the circuit. This inductance component varies depending upon the conductivity of the fluid in which the eddy current is induced. Blood has a different conductivity to other body fluids. The system is configured to detect presence of blood based on the additional inductance component. The system generates a data output representative of the determination.

As discussed, the invention is based on inductive sensing techniques.

Inductive sensing can be used as a means of non-invasive investigation of properties of a body.

Inductive sensing is based on generation of a primary alternating magnetic field via a primary antenna loop, which leads to the induction of eddy currents and a consequent secondary magnetic field in conductive material or tissue within the primary magnetic field. Interaction of the secondary magnetic field with the primary loop or the primary magnetic field can be used to detect patterns of movement within probed bodies, in particular those comprising a water content.

In particular, this field interaction leads to changes in the detectable electrical characteristics of the current running through the antenna coil. For example, the current frequency can be changed, and/or the current amplitude can be dampened.

In inductive sensing, a signal generator (such as an oscillator) is connected to a loop antenna. The oscillator is an amplifier, typically consisting of one or more transistors, which induces a resonant state in a coupled circuit, in combination with an inductance source and capacitance source. The inductance is provided by the loop antenna, while the capacitance is provided by an optional capacitor component placed in parallel to the loop, together with parasitic capacitances of the loop with itself and its environment, and the oscillator parasitic capacitances. The total system is called the resonator.

The secondary magnetic field generated by the body has the effect at the antenna loop of adding an additional complex inductance to the resonator circuit, which thereby leads to detectable changes in the circuit current. The real part of the additional inductance is detectable for example as changes in frequency of the oscillator circuit current. The imaginary part of the additional inductance is detectable for example as changes in amplitude of the oscillator circuit current (or voltage).

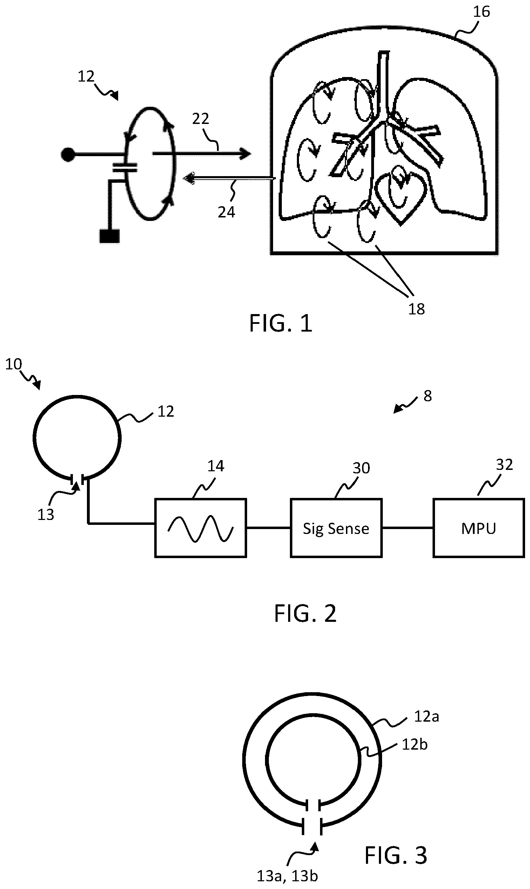

Inductive sensing is based on generation of a primary alternating magnetic field via a primary antenna loop, which leads to the induction of eddy currents and a consequent secondary magnetic field in conductive material or tissue within the primary magnetic field. This is schematically illustrated in Fig. 1. A loop antenna 12 of a resonator circuit is driven with an alternating current (drive signal). This causes current oscillations in the antenna.

In use, the antenna 12 is brought into proximity with a body 16 to be probed. The driving of the antenna generates primary electromagnetic (EM) signals 22 which couple with the body and generate eddy currents 18 in the body. These eddy currents depend on the conductivity of the body. The eddy currents generate a secondary magnetic field 24. This secondary field interacts with the primary field 22 to alter the oscillation characteristics of the resonator circuit.

In particular, in general, when a loop antenna is brought into proximity with a body, the inductance, L, acquires an additional reflected inductance component, $L_r$, arising due to the eddy currents 18 induced in the stimulated body as a result of application of the excitation signals 22.

These eddy currents 18 in turn effectively make a contribution to the inductance of the loop antenna 12, due to the generation of a secondary time-varying magnetic flux. These eddy-current fluxes combine with the primary flux of the antenna, resulting in a greater induced back-EMF in the antenna, and hence a larger measurable effective inductance.

The added component of inductance arising from the eddy currents may be referred to synonymously in this disclosure as 'reflected inductance'.

In general, the reflected inductance, $L_r$, is complex, and can be expressed as

$$L_r = L_r' + iL_r'' \qquad (1)$$

where $L_r'$ is related to a reactive impedance of the antenna and $L_r''$ is related to resistive impedance of the antenna.

The addition of the reflected component of inductance $L_r$ leads to a detuning of the characteristics of the antenna (or resonator circuit). In particular, both the natural radial frequency of the coil antenna circuit and the damping factor of the coil antenna circuit change.

In particular, the real part of the additional inductance component, $L_r$, manifests in the natural frequency of the resonator circuit or antenna. The imaginary part of the additional inductance component manifests in the (natural) amplitude of oscillations of the resonator circuit.

By way of completeness, it is noted that the circuit inductance is electrically related to the circuit impedance, and hence a measurement of real and imaginary parts of impedance may in some examples provide the required measured indicative of imaginary and real inductance. In particular, an impedance Z consists of a real and imaginary part: Z = R + iX, where R is the resistance and X the reactance. This can also be written as Z = R + iω*L, where ω is the radial frequency and L the inductance.

The reflected inductance, $L_r$, mentioned above can be defined as: Lr = Z/(iω) = L - iR/ω (= L' + iL"). L is in this case then called the real part of the reflected inductance (also denoted as L') and -R/ω the imaginary part of the reflected inductance (also noted as L").

Inductive sensing can also be used to distinguish between different fluids in a material. The electromagnetic signal response from different fluids varies depending upon their conductivity which in turn varies depending upon their physical properties. This provides a means for detecting presence of pools of blood at locations in the body where normally there should be no blood or only normal blood flow.

For example, in the cranial or intracerebral region, in a healthy patient, there should be a layer only of cerebrospinal fluid (CSF), along with normal blood flow through blood vessels in the area. In the case of intracerebral hemorrhage, accumulation or pooling of blood is present in addition. Blood and CSF have different conductivities. Thus where there is pooling or accumulation of blood, the average conductivity of the probed region changes, by virtue of there being a greater than normal concentration of blood in the area. Thus by measuring the inductive EM response at the intracerebral region (for example), it can be determined whether blood accumulation is present or only normal blood and CSF.

Similarly, for the abdominal region, for a healthy patient, there should be only abdominal or visceral fluid present, in addition to normal blood flow through blood vessels. Abdominal or visceral fluid has a different conductivity to blood. Thus the inductive response of application of the inductive sensor to the abdominal region permits presence of accumulations of blood to be detected, as compared to presence of only normal levels of blood plus abdominal or visceral fluid. As mentioned above, when there is abnormal bleeding and so pooling of blood, the average conductivity of the probed region changes, leading to a change in inductive response. This allows unusual accumulations of blood to be distinguished from normal blood flow in combination with other normal fluids.

Embodiments of the invention thus use inductive sensing to detect blood accumulations based on these principles.

Fig. 2 shows in block diagram form a sensing system in accordance with one or more embodiments of the invention.

The system 8 comprises a resonator circuit 10 comprising: a loop antenna 12 and an electronic signal generator 14 coupled to the antenna, for driving the antenna with a drive signal to cause it to generate the electromagnetic (EM) excitation signals. The signal generator in this example is in the form of an oscillator 14 which generates the drive signal with the drive frequency.

The resonator circuit 10 further includes in this example a capacitor 13 for setting or tuning a natural free space resonance frequency of the resonator circuit (i.e. natural frequency in the absence of any applied fields). The capacitor may in some examples be a variable capacitor to allow a natural free space resonance frequency to be adjusted.

The system 8 further comprises a signal sensing means 30, arranged for sensing, simultaneously with signal generation, the returned signals from the body based on detecting a measure indicative of an additional (complex) inductance component added to the antenna 12 of the resonator circuit 10 by said returned signals.

The secondary EM signals from the body add an additional (complex) inductance component to the circuit, as discussed above. This can be detected based on detecting changes to one or more electrical characteristics of the resonator circuit, for example by detecting changes in a natural damping factor of the resonator circuit and/or in a natural frequency of the resonator circuit. These may be detected respectively based on detecting changes to an amplitude of oscillations in the resonator circuit current or based on detecting changes to a frequency of oscillations in the resonator circuit for example. The additional inductance component will vary in dependence upon properties of the medium being probed (as discussed above).

The system is further configured to determine presence or absence of accumulation of blood based on the detected measure of the additional inductance component.

The system is further configured to generate a data output indicative of a result of the determination.

In some examples for instance, the determination of presence or absence of blood may be based on one or more pre-defined thresholds, the one or more thresholds associated with presence of bleeding in at least one of said one or more regions of the body. In this way bleeding can be detected.

However, use of a threshold is not essential. Alternative approaches include for instance use of an algorithmic model which is configured to determine presence or absence of blood in one or more particular regions based on the detected additional inductance component. Other alternative approaches might also include use for instance of artificial intelligence or machine learning algorithms, for instance trained for classifying between normal levels of blood in a region and abnormal pooling or accumulation of blood in a region. For example, a machine learning algorithm might be trained with training data, the training data comprising different additional inductance component signals for different regions, each labelled as to whether it corresponds to a normal blood presence or to abnormal accumulation or pooling of blood.

The above represent example approaches only and embodiments of the invention are not limited thereto.

The system may comprise a controller or processor configured to perform said determining and generating steps in some examples.

In the example of Fig. 2, the system 8 further comprises a microprocessor 32 arranged operatively coupled to the signal sensing means 30 and the resonator circuit 32. For example, the microprocessor may be configured for controlling a drive frequency of the drive signal generator 14, and/or it may be configured to perform signal processing, for example for said determining of presence or absence of bleeding. It may in some examples perform further signal processing, for example for deriving one or more physiological or anatomical parameters from a sensing or measurement signal detected by the signal sensing means 30.

The signal sensing means 30 can take different forms and operate in different ways for deriving said measure indicative of an additional inductance component added to the antenna of the resonator circuit by said returned signals.

The inductive sensing system may be configured in either a single-antenna setup or a multi-antenna setup.

In a single antenna setup, the resonator circuit comprises only a single antenna, preferably with a single loop.

In a multi-antenna setup the resonator circuit comprises two or more antennas, each driven with a drive signal to generate excitation signals, and wherein an additional inductance component added to each respective antenna is sensed by the signal sensing means.

Fig. 3 schematically illustrates an example multi-antenna configuration. In this example, two antennas are provided, a first 12a larger diameter antenna, and a second 12b smaller antenna diameter. In the illustrated examples, the two antennas are arranged concentrically, with the smaller antenna 12b inset (for example co-axially) inside the circumference of the larger antenna 12a. A support structure, e.g. a frame or housing, may be provided arranged to hold the two (or more) antennas in fixed relation to one another.

In this example, the two antennas are in co-planar arrangement, i.e. lying in the same plane. However, in other examples, the two or more antennas may be mounted axially offset from one another (i.e. offset in a direction perpendicular the plane defined by at least one of the antennas).

Furthermore, a concentric arrangement is not essential. In further examples, the second antenna 12b may be arranged outside of the circumference of the first antenna 12a, or its internal loop surface area may overlap with that of the first antenna.

Furthermore, according to one or more examples, the two or more antennas may each be driven with a drive signal of a different respective frequency. This will be discussed further below.

The single and multiple antenna configurations will now be described in more detail.

A single antenna 12 may be used to measure for example the reflected inductance at a specific location on the skull or abdominal region. For example a single loop placed on the skull may be used to detect intracerebral hemorrhage. A single loop placed on the abdomen may be used to detect abdominal bleeding, e.g. caused by abdominal trauma. An example will be considered below pertaining to use for detection of intracerebral hemorrhage

Based on the tissues that are in proximity to the loop antenna 12, the geometry of the loop and the position of the loop relative to the body (e.g. distance to the body), the value of the reflected inductance will vary. The term 'reflected inductance' has been introduced above and refers to the additional component of inductance added to the resonator circuit by secondary electromagnetic (EM) signals returned from the body responsive to application of the EM excitation signals.

Since blood is more conductive compared to cerebrospinal fluid (CSF) or abdominal and visceral fluid, the presence of blood will result in a higher reflected inductance. By measuring the exact value of the reflected inductance, it can be determined whether there is more blood present than would normally be expected, or if blood is present where it should not be present. In this way, inductive sensing can be used to detect internal bleedings in the abdominal region or brain.

For example, according to one set of possible embodiments, a pre-determined detection threshold may be defined in respect of the reflected inductance component, the threshold known to be associated with presence of bleeding, i.e. of abnormal accumulation or pooling of blood in the probed anatomical region. The system may be configured to compare the detected reflected inductance component obtained with the single loop with the threshold to determine whether there is bleeding or no bleeding (abnormal blood pooling, or normal amounts of blood). The value of the threshold may vary depending upon the region of the body under consideration. For example a different threshold may be defined for detecting intracerebral bleeding compared with detecting abdominal bleeding. The threshold may be determined in advance for example based on empirical measurements, or based on use of a model or simulation.

It is noted that in some cases a threshold may not be needed, and a model or simulation can be applied directly to determine presence or absence of blood accumulation, for instance by feeding the measured additional inductance component as an input to the model or simulation.

To illustrate the principle of blood detection with a single loop arrangement, an example simulation has been run by the inventors for detection of an intracerebral hemorrhage.

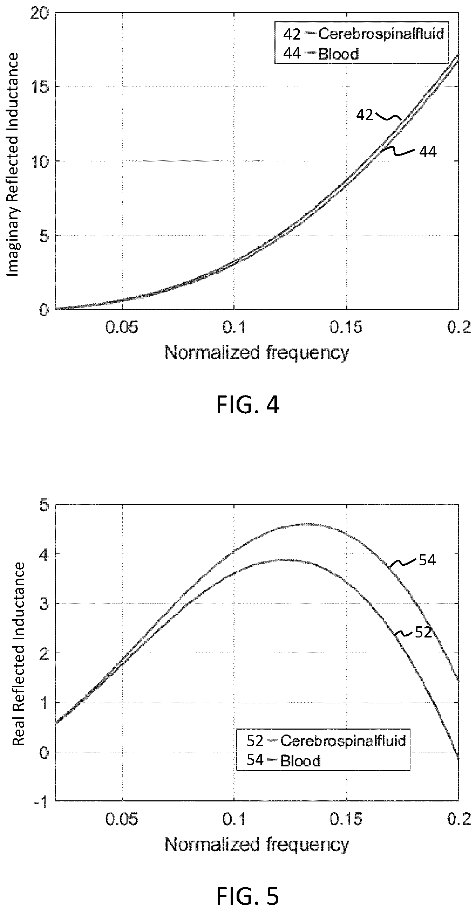

Figs. 4 and 5 show the difference in the imaginary (y-axis of Fig. 4) and real (y-axis of Fig. 5) part respectively of the reflected inductance as a function of (normalized) frequency (x-axis of Fig. 4 and 5) when a thin layer of blood or CSF is present behind the skull. Each graph shows the result for both blood and CSF. In Fig. 4, line 42 shows the result for CSF, and line 44 shows the result for blood. In Fig. 5, line 52 shows the result for CSF, and line 54 shows the result for blood.

In each graph, the frequency is normalized relative to the loop diameter. This normalization approach is discussed in detail in WO 2018/127482.

From the graphs of Figs. 4 and 5, it is clear that the presence of blood behind the skull results in an observably higher real component of reflected inductance 54 (Fig. 5) compared to the result 52 for CSF. A difference between the results for blood 44 and CSF 42 is also observable in the imaginary component of the reflected inductance (Fig. 4), but the result difference is smaller. Hence, for a single antenna setup, detecting the real component of reflected inductance may provide more reliable and robust blood accumulation detection results.

In advantageous examples for instance, the system 8 may be configured to detect an additional real inductance component added to the resonator circuit, and to determine whether this exceeds a pre-determined threshold, where the pre-determined threshold may be based on a known value (or value as a function of frequency) detected for a normal fluid such as CSF, or for a known normal level of blood flow in a given region in addition to CSF.

As the real component of inductance is related to the imaginary component of impedance, alternatively, in some advantageous embodiments, the system 8 may be configured to detect an additional imaginary impedance component added to the resonator circuit, and to determine whether this exceeds a pre-determined threshold where, the pre-determined threshold may be based on a known value (or value as a function of frequency) detected for a normal fluid such as CSF, or for a known normal level of blood flow in a given region in addition to CSF.

The layer model used for the simulations in this example consist of the following layers and corresponding thicknesses (in mm):

*Table 1*

| Layer | Thickness (mm) |
|---|---|
| Bone - cortical | 2 |
| Bone - cancellous | 3 |
| Bone - cortical | 2 |
| Cerebrospinal fluid / Blood | 1 |
| Grey matter | 5 |
| White matter | 100 |
| Air | infinite |

The loop was assumed to be placed at a distance of 3 mm from the first layer of cortical bone, which corresponds to a realistic value of for example a probe housing outer wall, which would be located between the loop and the skull.

The multiple-antenna arrangement will now be discussed.

According to one or more embodiments, a multi-loop setup may be provided comprising two or more antennas, preferably each consisting of a single loop or winding. Each may be provided with a different geometry (e.g. diameter, shape), position (e.g. distance) relative to the body and/or may be driven with a different drive frequency.

A multi-loop setup can be used for detecting bleeding in any region of the body including for example for detection of intracerebral hemorrhage and for abdominal bleeding.

When combining the measurement results of more than one antenna, the measurement becomes less sensitive for small changes in the tissue layers in proximity to the loops. For example, if the fat layer of the abdominal wall differs from patient to patient, the reflected inductance measured with a single antenna will change due to the increased or decreased distance of the layer of blood to the loop. However, with a multiple-loop setup, the measurement result of both antennas will change but the ratio between the two measurement results will remain more or less stable. Therefore, the accuracy of the blood presence detection increases.

This also advantageously means that calibration of the system between different patients is not required since, while the absolute measured values for the additional inductance (at a single antenna) may vary from patient to patient, the ratio between values of two loops is relatively stable across a majority of patients. Thus accuracy is improved without the need for patient-specific calibration.

Similarly, when a multi-loop setup is used to detect intracerebral hemorrhage, variations in the thickness of the skull have minimal influence on the ratio of reflected inductance, whereas the result with a single loop may vary.

Thus, in a multi-loop arrangement, the system may be configured to detect an additional inductance component added to each one of the plural antennas by said returned signals from the body.

In some embodiments, the system may further be configured to determine a ratio between the additional inductance components added to each of the two antennas, and compare said ratio with a pre-defined threshold (for the ratio) to determine presence or absence of blood. In other examples, a difference between the inductance components added to each of the two antennas may be determined, and this compared to a pre-defined threshold for the difference. More generally, comparative values of the additional inductance components of the two antennas may be used for the determination.

A pre-determined threshold may be defined pertaining to the ratio of reflected inductance component values between the two (or more) loops. The ratio may be specific to the loop arrangement. For example, it may correspond to the ratio of the smaller loop 12b to the larger loop 12a. Ratio in this context means the quotient of the reflected inductance component values for the two loops. The ratio threshold may be a ratio known to be associated with presence of (abnormal) accumulation or pooling of blood.

The ratio threshold may be pre-determined based on empirical measurements, or may be based on a model or simulation for example.

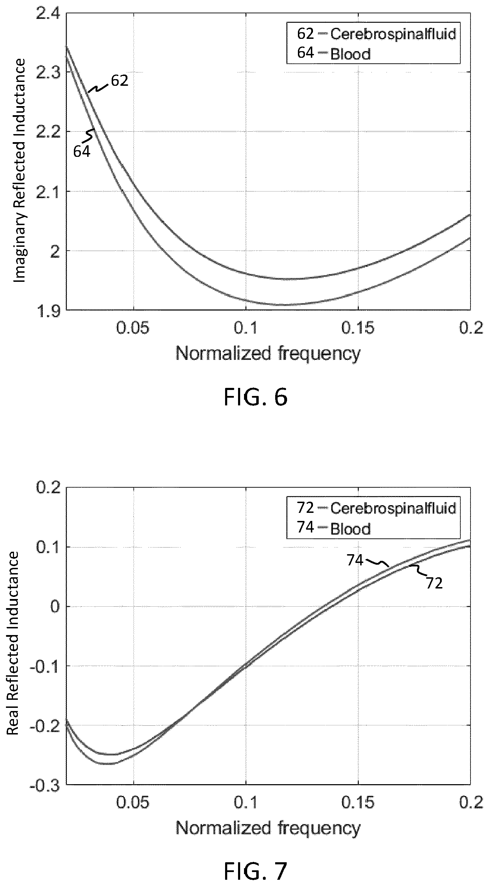

To illustrate the multiple antenna setup, a further simulation has been run by the inventors, the results of which are shown in the graphs of Figs. 6 and 7. The simulation was run with the same tissue layers as used in the simulation of the single-loop setup (i.e. as indicated in Table 1 above). The setup consisted of two concentric loops (e.g. as shown in Fig. 3) with a diameter of 10 mm and 20 mm respectively, and configured in co-planar arrangement so that a distance from the antenna to skull is the same for both antennas. These diameter sizes are by way of example only, and in further examples, any other antenna diameter values may instead be used.

The ratio between the measured reflected inductance of these two loops is plotted for the imaginary (y-axis of Fig. 6) and real parts (y-axis of Fig. 7) of the inductance as a function of frequency (x-axis of Fig. 6 and 7). The ratio in each case corresponds to the inductance value of the smaller loop (loop 1) divided by the inductance value of the larger loop (loop 2), i.e. the quotient of the smaller loop to the larger loop.

Each graph shows the ratio results for both CSF and for blood. In Fig. 6, line 62 shows the result for CSF and line 64 shows the result for blood. In Fig. 7, line 72 shows the result for CSF and line 74 shows the result for blood.

In this case, the greatest difference between the blood and CSF results is found in the ratios for the imaginary components of reflected inductance (Fig. 6). Here, the ratio between the imaginary reflected inductance components of the two loops (loop 1 / loop 2) is observably smaller for blood than for CSF.

Thus in some embodiments, the system 8 may be configured to extract the imaginary component of the reflected inductance component added at each antenna and to determine the ratio or quotient between these imaginary components only. Working with the imaginary component for the ratio may result in more reliable or accurate detection results.

Also, since the imaginary component of inductance is related to the real component of impedance, alternatively, in some advantageous embodiments, the system 8 may be configured to extract the real part of an additional component of impedance added at each antenna and to determine the quotient or ratio between these real components of impedance.

However, a difference is also observable between the ratio results in the real components of reflected inductance (Fig. 7). Hence in further embodiments, the ratio of the real components of reflected inductance may alternatively or additionally be used in the multi-loop arrangement.

Although an arrangement has been discussed comprising only two antennas (two loops), in further embodiments, more than two antennas (two loops) may be provided. In some embodiments, detection of blood accumulation may be based on relative values of the additional inductance components added to each of the three of more antennas. For instance a difference between the inductance components for different pairs of the three of more loops may be calculated, or an average difference between the loops may be calculated or a quotient between the inductance components for the three of more loops may be calculated. In some examples, a difference between the inductance components for various pairs of the three or more antennas may be calculated, and a common baseline or offset known to be present in each subtracted (e.g. representative of a skull thickness). This may allow for comparison values to be more reliably indicative of difference in blood amounts in the different regions.

Furthermore, although the arrangement above comprises concentric and co-planar antennas, in further embodiments, the antennas may be provided axially offset from one another and/or non-concentrically arranged.

According to one or more embodiments, the two or more loops may be driven with drive signals of different respective drive frequencies. This may further enhance robustness of measurement, since different frequencies will vary to differing degrees as a function of changing layer thicknesses. Hence a ratio of reflected inductance for two loops driven at different frequencies will tend to be more consistent between different patients than a two-loop arrangement in which both loops are driven with the same frequency.

According to one or more embodiments, one or both loops may be driven with a time-varying frequency. For example, the frequency may be varied in a continuous fashion (i.e. a frequency sweep performed), and/or the frequency may be changed in discrete steps. The strength of signal response (at the desired measurement depth) may vary depending upon the drive frequency. Hence by executing a frequency sweep, the optimum frequency (offering strongest signal response) may be identified.

As discussed above, for either the single or multiple antenna arrangements, according to one group of embodiments, a threshold may be used for the blood detection. The value of the threshold may vary depending upon the region of the body under examination. For example, there may be a different threshold for the intracerebral region compared to the abdominal region. As also discussed above, use of a threshold is not essential and other algorithm or model or machine learning based detection approaches are possible for example.

In accordance with one or more embodiments, the system may be switchable between at least two detection modes:

a first detection mode in which the system is configured for detecting presence of accumulation of blood in an intracerebral region of the body; and

a second detection mode in which the system is configured for detecting presence of accumulation of blood in an abdominal region of the body.

Both the single and multiple-loop setups can be embodied in different ways.

In either case, a support structure may be provided for holding the one or more antennas of the resonator circuit in a fixed position, for example in fixed relation relative to one another. Various example support structures will now be outlined.

In accordance with one or more embodiments, the sensing system may comprise a handheld probe unit, the handheld probe unit comprising at the least the resonator circuit. The handheld probe in this case may comprise a housing or frame which provides a support structure.

A universal handheld device with a single loop or multi-loop setup could be used to probe both the skull and torso (as well as any other desired body region). There may be provided a means for inputting to the device the anatomical region with which it is being used, e.g. skull or torso, or other region. Where a threshold is used for performing the detection of blood pooling, it may then configure threshold settings based on this input. The device may be provided means for automatically detecting the region it is sensing, e.g. a location sensor. Alternatively, there may be a user input means for providing this information to the probe. This could for example be a simple switch for switching between different modes for different regions, e.g. skull-mode or torso-mode. This may in some examples be a 'software switch' implemented by the control unit or processor of the device or system.

In some examples, the system may be configured to automatically select the mode based on detected inductance signal values at the region at which the probe is placed. For example an initialization phase may be triggered for automatically configuring the right mode. The handheld unit is placed at the anatomical region to be probed and the initialization phase activated. The system begins generating inductive sensing signals, and based on values for instance of the additional inductance component measured at the antenna, it can be determined which anatomical region is being probed. For example a lookup table may be stored which records typical ranges of inductive signal (e.g. additional inductance component) values for different anatomical regions. Based on consulting such a table, the anatomical region location of the device can be estimated, and thus the correct mode for that region automatically selected.

According to a further set of embodiments, the system 8 may comprise a head wearable cap, the at least one antenna being mounted to the cap. There may preferably be provided multiple antennas 12 mounted to the cap.

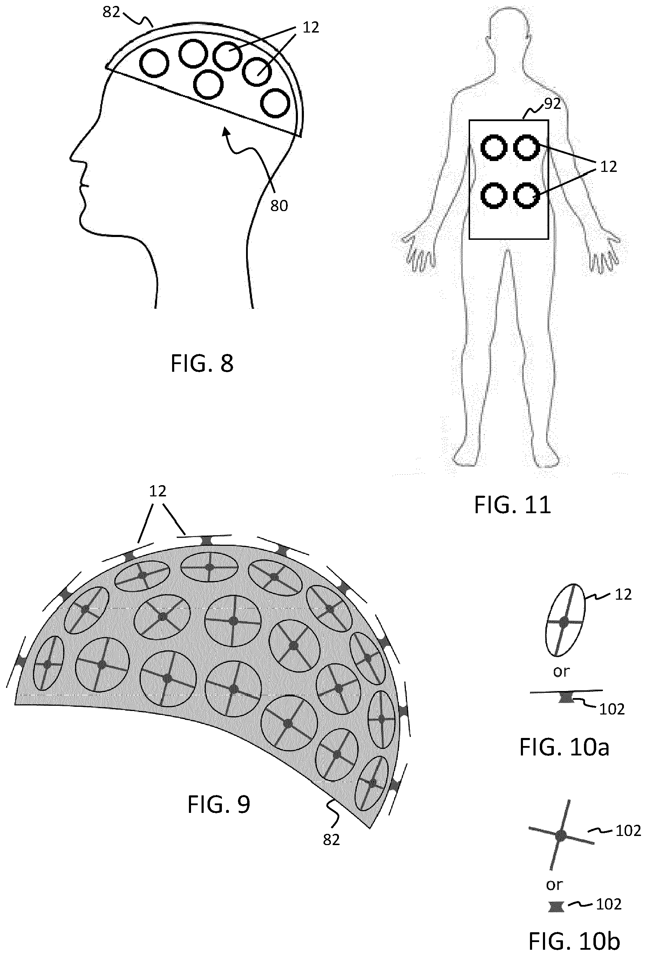

An example is shown schematically in Fig. 8. Fig. 8 shows an example head cap 82 comprising multiple antennas 12 mounted or coupled to an outer surface of the cap (i.e. a surface furthest from the head when worn). The hat or cap may be placed on the patient head in use and the single or multiple antenna loops measure the reflected inductance at multiple locations on the skull. This confers the benefit that clinical staff are not required to hold the device. This may be beneficial in for example an ambulance. The cap can also ensure that the antennas are held in a stable position relative to the head, and at for instance fixed uniform distance from each other. This may be beneficial for reliable and comparable measurement results between testing instances and between patients.

The cap can be configured in such a way that the position of the antennas is fixed relative to the head and that the separation or spacing of each inductive sensor relative to the skull is uniform over the full skull.

This can be done for example by using a flexible cap so as to ensure maintenance of contact with the skull (the cap is head retained around the head when worn). Optionally, multiple holes may be delimited by the cap so that cap takes the form of a net or mesh.

In preferred examples, each single or multi-antenna may be connected to the flexible cap or flexible net at a single point. Thus, even though the loop is rigid, the cap itself is fully flexible.

This is shown in Fig. 9 and Fig. 10a and 10b. Fig. 9 illustrates an example cap 82 and shows an arrangement of multiple single antennas 12 mounted to the cap, each via a single connection point 102. Each antenna is mounted to an outer surface of the cap or net via a spacer member 102 which holds each antenna upstanding from, or held elevated above, an outer surface of the cap, hat or net (i.e. a surface facing away from the head). Each spacer member takes the form of a stem or rod holding a respective antenna elevated above the cap outer surface.

The spacer 102 or stem ensures that the center of the antenna loop 12 circumference is maintained a constant distance from the outer surface of the cap. In further examples, the spacer may be provided in the form of a cladding surrounding the wire of the antenna.

The array of inductive sensor antennas shown in Fig. 9 permit generation of a map of the conductivity of the skull, for example in the top 5-10 cm of the skull. Intracerebral hemorrhage at the surface of the skull may then be detected as a local increase of the conductivity for example.

In in advantageous set of examples, the cap 82 may contain a visual indictor means, and wherein the system is configured to control the visual indicator means to provide a visual indication of the determination of presence or absence of bleeding in the cerebral (e.g. intracerebral) region.

This indicator may for example comprise a red-green light or display attached to the cap 82, e.g. red indicating bleeding, green indicating no bleeding.

The cap may be operably connectable in use, e.g. with either a wired or wireless connection, to a readout device or patient monitor.

Fig. 10 shows an example antenna 12 and coupled spacer 102 or stem (the single-point connector). Fig 10b illustrates views of the spacer 102 or stem by itself.

In accordance with a further set of embodiments, the system may comprise a (e.g. textile) garment or sheet for covering one or more regions of the body to be inductively sensed, where the at least one antenna 12 is mounted to the garment or sheet.

Preferably the system comprises a plurality of antennas, mounted to the garment or sheet at different positions.

Such an arrangement is particularly advantageous for example for performing inductive sensing of the torso, e.g. abdomen.

According to one or more examples for instance a blanket or vest may be provided which may be placed on the patient, the blanket or chest comprising a plurality of antennas 12 mounted thereto.

An example is shown schematically in Fig. 11. This shows an example blanket article 92 comprising an array of antennas 12 mounted across its area. These may be mounted on an outer surface of the blanket or may be integrated in the body of the blanket, for example sewn or zipped inside.

The blanket 92 may be placed on the patient during a measurement for example, enabling the medical staff to focus on other tasks.

In in advantageous set of examples, the blanket may contain a visual indictor means, and wherein the system is configured to control the visual indicator means to provide a visual indication of the determination of presence or absence of bleeding in the abdominal region.

This indicator may for example comprise a red-green light or display attached to the blanket, e.g. red indicating bleeding, green indicating no bleeding.

In one set of embodiments, the visual indicator means may be arranged to detect blood accumulation across a plurality of regions of the patient's body which may be covered by the garment or sheet, e.g. blanket. The visual indicator means may comprise a plurality of visual indicator parts arranged to provide a visual indicator at different locations of the garment of sheet to indicate corresponding bodily locations of detected blood pooling. For example, the visual indicator means may comprise a plurality of light sources disposed at different locations across the sheet or garment, and wherein the system is configured to illuminate light sources at locations aligned with (e.g. situated above) any location of the body at which the plurality of antennas of the garment or vest detect blood accumulation.

For example, the position of the garment or vest on the patient may be landmarked, for instance the center of the top edge of a blanket article positioned at the suprasternal notch or the xiphoid. A clinician when positioning the garment or vest on the patient could ensure the reference point on the garment or vest correctly lines up with the pre-defined reference alignment location on the patient's body. In this way, the article is positionally calibrated.

A similar feature could also be applied to the head-wearable cap 82 discussed above, i.e. the head wearable cap may comprise a visual indicator means arranged to provide a visual indicator at different locations of the cap to indicate corresponding cerebral locations of detected blood pooling.

The blanket may be operably connectable in use, e.g. with either a wired or wireless connection, to a readout device or patient monitor.

Multiple antenna loops 12 may be used, distributed across the area of the blanket, for measuring multiple locations at once without having to hold any device. The same single point attachment as shown and described for the flexible cap 82 above may also be used for this embodiment.

Embodiments of the present invention thus provide an inductive sensor which can be used in almost any environment to detect presence of bleeding. Advantages of the provided system include that the system may be provided in a compact form factor, e.g. embodied as a handheld device, a blanket or a small cap as discussed above. This means it may be readily used for a pre-hospital or emergency triaging setting. Additionally, the result is instantaneous and easy to interpret. In particular, the data output signal of the inductive sensor system will immediately represent any bleeding by means of a difference in reflected inductance. Therefore, initial triaging (e.g. for ICH or abdominal bleeding) can be done anywhere with low levels of operator expertise and in a timely way.

Examples in accordance with a further aspect of the invention provide an inductive sensing method for detecting presence of bleeding in one or more regions of a patient's body, the method based on sensing electromagnetic signals returned from the body responsive to application of electromagnetic excitation signals to said body.

Fig. 11 shows a block diagram of steps of an example method 100 in accordance with one or more embodiments.

The method comprises driving 102 at least one loop antenna of a resonator circuit with a drive signal to cause it to generate the electromagnetic excitation signals.

The method further comprises sensing 104, simultaneously with the signal generation, said returned signals from the body based on detecting a measure indicative of an additional inductance component added to the antenna of the resonator circuit by said returned signals.

The method further comprises determining 106 presence or absence of blood based on the detected measure of the additional inductance component.

The method further comprises generating 108 a data output indicative of a result of the determination.

The method may be for detecting presence of blood in a cranial region. The method may be for detecting presence of blood in an abdominal region. The method may further comprise determining, based on blood detection, occurrence of either intracerebral hemorrhage, or abdominal bleeding.

For example, the method may comprise positioning the at least one antenna of the resonator circuit for applying electromagnetic excitation signals to the head region or the abdominal region, and receiving returned electromagnetic signals from the head region or abdominal region.

As discussed above in relation to the system aspect of the invention, the determination of presence or absence of blood accumulations may be based on a pre-defined threshold, the threshold associated with presence of blood in at least one of said one or more regions of the body.

The threshold may be a threshold known to be associated with presence of bleeding in the cranial region or the abdominal region as appropriate.

Implementation options and details for each of the above steps may be understood and interpreted in accordance with the explanations and descriptions provided above for the apparatus aspect of the present invention (i.e. the system aspect).

Any of the examples, options or embodiment features or details described above in respect of the apparatus aspect of this invention (in respect of the inductive sensing system) may be applied or combined or incorporated into the present method aspect of the invention.

Examples in accordance with a further aspect of the invention may provide a computer program product comprising code means configured, when executed on a processor, the processor bring operatively coupled to a

a resonator circuit 10 comprising at least one loop antenna 12, and an electronic signal generator 14 coupled to the antenna, for driving the antenna with a drive signal to cause it to generate the electromagnetic excitation signals, and a signal sensing means 30 arranged for sensing, simultaneously with signal generation, said returned signals from the body based on detecting a measure indicative of an additional inductance component added to the antenna of the resonator circuit by said returned signals

to cause the processor to perform an inductive sensing method in accordance with any embodiment outlined above or described below, or in accordance with any claim of this application.

As discussed above, some embodiments make use of a control means and/or a microcontroller and/or microprocessor.

Any or each of these components may be implemented in numerous ways, with software and/or hardware, to perform the various functions required. For example a processor may be used which employs one or more microprocessors that may be programmed using software (e.g., microcode) to perform the required functions. A control means may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. An inductive sensing system (8) arranged for sensing electromagnetic signals returned from a body responsive to application of electromagnetic excitation signals to said body, the system adapted to detect presence of bleeding in one or more regions of the body, the system comprising:

    a resonator circuit (10) comprising at least one loop antenna (12), and an electronic signal generator (14) coupled to the antenna, for driving the antenna with a drive signal to cause it to generate the electromagnetic excitation signals; and
    a signal sensing means (30) arranged for sensing, simultaneously with signal generation, said returned signals from the body based on detecting a measure indicative of an additional inductance component added to the antenna of the resonator circuit by said returned signals;
    the system configured to:

    determine presence or absence of accumulation of blood based on the detected measure of the additional inductance component; and
    generate a data output indicative of a result of the determination.

2. The inductive sensing system of claim 1, wherein the determining the presence or absence of blood accumulations is based on a pre-defined threshold, the threshold associated with presence of bleeding or blood accumulation in at least one of said one or more regions of the body.

3. The inductive sensing system of claim 1 or 2, wherein the resonator circuit (10) comprises only a single antenna (12), the antenna comprising a single loop, and
preferably wherein the signal sensing means (30) is configured to detect a real component of said additional inductance component added to the resonator circuit.

4. The inductive sensing system (8) as claimed in any of claims 1-3, wherein the system is adapted to detect presence of blood accumulation in an intracerebral region of the body, and preferably wherein the system is further configured to detect occurrence of intracerebral hemorrhage based on the blood detection.

5. The inductive sensing system (8) as claimed in any of claims 1-4, wherein the system is adapted to detect presence of blood accumulation in an abdominal region of the body, and preferably wherein the system is further configured to detect presence of abdominal bleeding based on the blood detection.

6. The inductive sensing system (8) as claimed in claim 4 and 5, wherein the system is switchable between at least two detection modes:

    a first detection mode in which the system is configured to detect presence of blood accumulation in an intracerebral region of the body; and
    a second detection mode in which the system is configured to detect presence of blood accumulation in an abdominal region of the body.

7. The inductive sensing system (8) as claimed in any of claims 1-6, wherein the resonator circuit (10) comprises at least two single loop antennas (12a, 12b), and wherein the system is configured to
detect an additional inductance component added to each one of the antennas by said returned signals from the body.

8. The inductive sensing system (8) as claimed in claim 7, the system further configured to
determine a ratio between the additional inductance components added to each of the two antennas (12a, 12b), and preferably configured to compare said ratio with a pre-defined threshold to determine presence or absence of blood accumulation.

9. The inductive sensing system (8) as claimed in claim 7 or 8, wherein
the two loop antennas are arranged concentrically with one another;
the two loop antennas are mounted axially offset with respect to one another, and/or
the signal generator (14) is configured to drive the two antennas with drive signals of different respective AC frequencies.

10. The inductive sensing system (8) as claimed in any of claims 1-9, wherein the system comprises a support structure (82, 92), the at least one loop antenna (12) being mounted in fixed relation to the support structure.

11. The inductive sensing system (8) as claimed in any of claims 1-10, wherein the sensing system comprises a handheld probe unit, the handheld probe unit comprising at least the resonator circuit (10).

12. The inductive sensing system (8) as claimed in any of claims 1-11, wherein the system comprises ahead wearable cap (82), the at least one antenna (12) being mounted to the cap (82), such that, when the cap is worn, the at least one antenna is held in fixed relation to a surface of the head, and preferably wherein the system comprises a plurality of antennas (12) mounted to the cap at different positions, so as to be held at different locations about the head when the cap is worn, and
optionally wherein the cap (82) is formed of a flexible material so as to be held retained around the head when worn.

13. The inductive sensing system (8) as claimed in any of claims 1-10, wherein the system comprises a garment or sheet (92) for covering at least one of said one or more regions of the body, the at least one antenna (12) being mounted to the garment or sheet, and
preferably wherein the system comprises a plurality of antennas, mounted to the garment or sheet at different positions.

14. An inductive sensing method (100) for detecting presence of bleeding in one or more regions of a patient's body, the method based on sensing electromagnetic signals returned from the body responsive to application of electromagnetic excitation signals to said body, the method comprising:

driving (102) at least one loop antenna of a resonator circuit with a drive signal to cause it to generate the electromagnetic excitation signals;
sensing (104), simultaneously with the signal generation, said returned signals from the body based on detecting a measure indicative of an additional inductance component added to the antenna of the resonator circuit by said returned signals;
determining (106) presence or absence of accumulation of blood based on the detected measure of the additional inductance component, and
generating (108) a data output indicative of a result of the determination.

15. The inductive sensing method (100) of claim 14, wherein the method is for detecting presence of blood in a intracerebral region or in an abdominal region, and preferably wherein the method further comprises determining, based on blood detection, occurrence of either intracerebral hemorrhage, or abdominal bleeding.

FIG. 1

FIG. 2

FIG. 3

FIG. 4

FIG. 5

FIG. 6

FIG. 7

FIG. 8

FIG. 11

FIG. 9

FIG. 10a

FIG. 10b

100

Generate EM excitation signals — 102

Simultaneously sense returned EM signals from body — 104

Determine presence or absence of bleeding using a threshold and measured inductance — 106

Generate data output based on result — 108

FIG. 12

Europäisches
Patentamt

European
Patent Office

Office européen
des brevets

**EUROPEAN SEARCH REPORT**

Application Number

EP 20 15 0306

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JACOB GRIFFITH ET AL: "Non-Invasive Electromagnetic Skin Patch Sensor to Measure Intracranial Fluid-Volume Shifts", SENSORS, vol. 18, no. 4, 29 March 2018 (2018-03-29), page 1022, XP055665070, DOI: 10.3390/s18041022 * abstract * * 2.1. Sensor Design and Data Collection Setup; page 3 - page 4; figure 1 * * 3.1. Electromagnetic Resonant Sensor; page 8; figure 4 * * 2.2. Theory and Operating Principle; page 4 - page 5 * * Potential Clinical Applications; page 13 * * figure 8 * | 1-5,10, 11,14,15 | INV. A61B5/055 A61B5/02 |
| A | BLUMROSEN G ET AL: "New Wearable Body Sensor for Continuous Diagnosis of Internal Tissue Bleeding", WEARABLE AND IMPLANTABLE BODY SENSOR NETWORKS, 2009. BSN 2009. SIXTH INTERNATIONAL WORKSHOP ON, IEEE, PISCATAWAY, NJ, USA, 3 June 2009 (2009-06-03), pages 120-124, XP031522339, ISBN: 978-0-7695-3644-6 * abstract * | 1-6,10, 11,14,15 | |

TECHNICAL FIELDS SEARCHED (IPC)

A61B

-/--

~~The present search report has been drawn up for all claims~~

| Place of search | Date of completion of the search | Examiner |
|---|---|---|
| The Hague | 3 July 2020 | Weiss-Schaber, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 15 0306

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CÉSAR A GONZÁLEZ ET AL: "Remote Monitoring of Internal Bleeding Based on Magnetic Induction and Cellular Phone Technology: A Potential Application in Poor Regions in México", COMPUTACION Y SISTEMAS, vol. 14, no. 2, 26 January 2010 (2010-01-26), pages 187-195, XP055664989, MX ISSN: 1405-5546 * abstract * | 1-6,10, 11,14,15 | |

-----

TECHNICAL FIELDS
SEARCHED     (IPC)

~~The present search report has been drawn up for all claims~~

| Place of search | Date of completion of the search | Examiner |
|---|---|---|
| The Hague | 3 July 2020 | Weiss-Schaber, C |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt
European
Patent Office
Office européen
des brevets

Application Number

EP 20 15 0306

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

2-6, 10, 11, 14, 15(completely); 1(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

## LACK OF UNITY OF INVENTION
## SHEET B

Application Number

EP 20 15 0306

The Search Division considers that the present European patent application does not comply with the requirements of unity of invention and relates to several inventions or groups of inventions, namely:

```
1. claims: 2-6, 10, 11, 14, 15(completely); 1(partially)

        Inductive detection of internal bleeding using only a single
        antenna with a single coil
                         ---

2. claims: 7-9(completely); 1(partially)

        Inductive detection of internal bleeding having at least two
        single loop antennas
                         ---

3. claims: 12, 13(completely); 1(partially)

        Wearable item comprising inductive sensing system
                         ---
```

**EP 3 845 124 A1**

**Patent documents cited in the description**

- WO 2018127482 A